# EUROPEAN PATENT SPECIFICATION

(11) **EP 1 219 625 B1**
(45) Date of publication and mention of the grant of the patent: **17.05.2006**
(21) Application number: 00128571.7
(22) Date of filing: 27.12.2000
(51) Int. Cl.: C07D 513/04

(54) **Novel substituted 2-(6-benzyl-5-oxo-3-phenyl-(S3,7S,7AR)- perhydroimidazol (1,5-C) (1,3) thiazol-7YL) compounds**
Neue substituierte 2-(6-Benzyl-5-Oxo-3-Phenyl-(S3,7S,7AR)-Perhydroimidazol- (1,5-C) (1,3)- Thiazol-7-YL Verbindungen
Neue composés substituées de la 2-(6-benzyle-5-oxo-3-phényl-(S3,7S,7AR)- perhydroimidazol (1,5-C) (1,3)- thiazol-7-YL

(43) Date of publication of application: 03.07.2002
(73) Proprietor: COUNCIL OF SCIENTIFIC AND INDUSTRIAL RESEARCH, New Delhi 110 001 (IN)
(72) Inventor: Chavan, Subhash, Prataprao, Maharashtra (IN); Chittiboyina, Amar, Gopal, Maharashtra (IN); Kamat, Subhash, Krishnaji, Maharashtra (IN); Kalkote, Uttam, Ramrao, Maharashtra (IN); Ravindranathan, Thotapallil, Maharashtra (IN)
(74) Representative: Viering, Jentschura & Partner

(56) References cited:
- EP-A- 0 469 432
- US-A- 4 694 087
- US-A- 4 732 987
- US-A- 5 233 045

## Description

The invention relates to novel substituted 2-[-6-benzyl-5-oxo-3-phenyl-(3s,7s,7aR)-perhydroimidazol[1,5-c][1,3]thiazol] compounds having general formula (7) 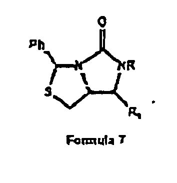 Wherein R= benzyl, R1 = alkyl group selected from 1-phenyl -1-ethanone, 1-(4-chlorophenyl)-1-ethanone, 1-(4-methoxypheny)-1-ethanone,2-oxocyclohexyl, 1-trimethylsilyloxy-2-oxocyclohexyl, allyl,1-hexanyl,4-dimethylaminophenyl, or 2-methylpropanoate. Still more particularly it relates to methyl 6-[benzyl-5-oxo-3-phenyl- (3S, 7aR)-perhydroimidazo[1,5-c][1,3]thiazol-7yl]-6-oxohexanoic acid of formula (I) which is very useful intermediate for D(+)-Biotin

### BACKGROUND ART

Biotin (Vitamin H) is one of the B-complex group of vitamins and has immense commercial importance in the area of animal health and nutrition. It is one of the biocatalysts of the reversible metabolic reactions of carbon dioxide transport in micro and macro organisms. It is used in poultry feeds of rapid growth of chicks and healthy hatching of eggs. Biotin avidin complex finds a vital role in the area of biochemistry. D(+)-Biotin prepared by the process of the present invention is represented by formula 2

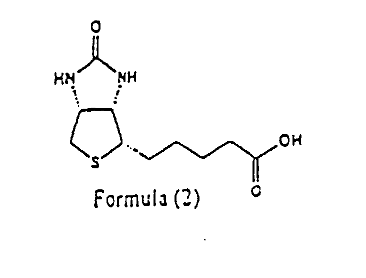

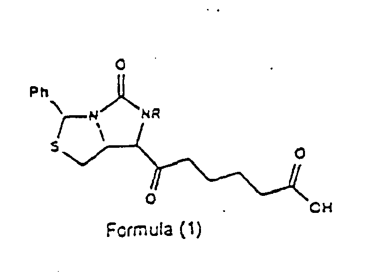

D(+) Biotin is prepared in the prior art from amino acids *viz.,* cysteine, cyctine and serine. These processes involving L-cystine as the precursor, incorporate intramolecular radical cyclization (E.J.Corey, M.M.Mehrotra, *Tet.Lett*., 29, 57 1988) as the key step is construct the tetrahydrothiophene moiety of biotin.

Another prior art process involves intramolecular cycloaddition (3+2) of derivatives of L-cystine (E.G.Baggiolini, H.L.Lee, G.Pizzolato and M.R.Uskokovic, *J.Am.Chem.Soc*., 104, 6460, 1982), and L-cysteine (, H.L.Lee, E.G.Baggiolini and M.R.Uskokovic, *Tetrahedron* 43, 4887, 1987).

In another process starting from L-cysteine, a bicyclic imidazolidine is the key intermediate leading to D(+)-biotin (E.Poetsch and M.Casutt, EP 242,686 1986 CA:108:112077k 1988; Chimia 41, 148 1987). In a totally different and novel approach. L-cysteine was converted to its thiazolidine derivative which on treatment with bromine is converted stetreospecifically to a bicyclic intermediate as a single stereoisomer and eventually transformed to D(+)-biotin (P.N. Confalone, E.G. Baggiolini, D. Lollar, and M.R. Uskokovic, *J. Am. Chem. Soc., 99,* 7020 1977).

Process for stereospecific synthesis of D(+)-Biotin from sugars of suitable configuration are known. [From Mannose *Tet. Lett., 32*, 2765 1975].

The use of L-cysteine in known from US Patent 4000972, US 4130713, US 433 7345 and *J. Am. Chem. Soc., 99,* 7020, 1977 avoids the handling of labile intermediate steps but involves 18 steps, in all with the separation of undesired isomer leading to unsatisfactory yields of optically active D(+)-Biotin.

In another process *J. Am. Chem. Soc.,* 105, 5946, 1983 and EP 0094776, substituted 3H, 5H-Imidazo [1,5-C]tetra hydro thiazole are described from which after racemate resolution, synthesis of optically active biotin is described.

In another process of Moolenaer, M. J.; Speckamp, W.H.; Hiemstra, H.; Poetsch. E.; Casutt, M. *Angew. Chem., In. Ed. Engl*. 1995, 34, 2391 and DE 3,926,690 involves the intramolecular version of the condensation of a silyl enol ether with N-acyliminium intermediates to effect the ring closure of thio ether to the thiophane nucleus.

In yet another process of Poetsch, E.; Casutt, M.; EP 242,686 1986; CA: 108: 112077K 1988; *Chimia 41,* 141-150, 1987 describes the formation of keto acid and further elaboradtion of acid to D(+)-Biotin. The main draw back of this method is use of hazardous 2 eq of diisobutylaluminium hydride (DIBAL-H). Potassium cyanide (KCN), Carbonyldiimidazole and involves reactions to be performed under anhydrous conditions.

In yet another prior art of biotin synthesis by Chavan, S. P.; Chittiboyina, A.G.; Kamat. S.K. Indian Patents NF 135/98, 136/98 and Ravindranathan, T.; Chavan. S. P.; Tejwani, R.B. USP No.5,274,107 (1993) describes the formation of substituted imidazolidines from L-cysteine and further elaboration of hydantoin to D(+)-biotin.

The process also involves the use of expensive and hazardous chemicals viz., DIBAL-H, tertiary butyl dimethyl silyl chloride (TBDMSCI), trifluoromethane sulphonic acid (Triflic acid) etc.

Hitherto known processes involve highly toxic and hazardous chemicals e.g. phosgene for the formation of imidazolidine. Moreover the intramolecular radical cyclization leads to both desired five membered as well as undesired six membered ring along with tin inclusion compounds as the undesired byproducts.

In another prior art process involving the intramolecular (3+2)-cylcoaddition reaction of nitrone, the precursor olefin is obtained as a mixture (9:1) of which the desired olefin has to be purified and separated by chromatography. Moreover, the chiral intermediates obtained during the above mentioned sequence of reactions were prone to racemization.

In another prior art process involving the intramolecular cyclization of thiazolidine required Cbllins oxidation as one of the steps. Use of heavy metals on an industrial scale would lead to problems during waste disposal. Moreover Wittig reaction on the aldehyde leads to a mixture of isomers, which should be separated and the desired isomer subjected to further reactions leading to biotin.

All these processes are however characterized by large number of synthetic steps resulting in low overall yields. The non crystallizable intermediate stages mostly due to sugar nature are often obtained in impure form and require tedious purification on account of their polyfunctionality and chemical liability connected with it maintenance of comparatively very narrow range of reaction parameters. Additionally, these sugars are not easily available in nature, which leads to high prices.

Hitherto known processes involve highly toxic, expensive and hazardous chemicals for example carbonyldiimidazole. Methyl Iodide, potassium cyanide DIBAL-H etc. for the formation of keto acid (1). Moreover these reactions required anhydrous conditions.

US 5,233,045 discloses a process for the preparation of an imidazothiazolone derivative. For example, the preparation of 3-phenyl-6-benzyl-7-(5-carboxy-1-oxopentyl)-7,7a-dihydro-imidazo(1,5-c) thiazole is described which is prepared by reaction of a phosphorus ylide with an imidazothiazolone derivative.

An object of the present invention is to provide substituted [6-benzyl-5-oxo-3-phenyl-(3S,7S,7aR)-perhydroimidazo[1,5-c][1,3]thiazol]-7yl compounds of formula (7) especially as given table-1 which can be used for synthesis of D(+)-biotin of formula (2).

Another object of the present invention is to provide selective Baeyer Villiger oxidation and new methodology for nucleophilic addition to imidazolodine of the formula (6) at C-7 position for the synthesis of compound having formula (7).

### DETAILED DESCRIPTION OF THE INVENTION

Accordingly the present invention provides Substituted [6-benzyl-5-oxo-3-phenyl-(3S,7S,7aR)-perhydroimidazo[1,5-c][1,3]thiazol]-7-yl compounds having general formula (7). 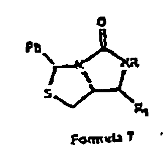 wherein R= benzyl, Rₜ= alkyl selected from 1-phenyl-1-ethanone, 1-(4-chlorophenyl)-1-ethanone, 1-(4-methoxyphenyl)-1-ethanone, 2-oxocyclohexyl, 1-trimethylsilyloxy-2-oxocyclohexyl, allyl, 1-hexynyl, 4-dimethylaminophenyl, or 2-methylpropanoate.

The present invention also provides a process for the preparation of compounds having formula (7) which comprises reacting the compound of formula (6) with Lewis acid and a nucleophile at a temperature ranging between 0° to 3°C in an organic solvent for 10-30 minutes quenching the reaction mixture with either water or saturated aqueous solution of quenching agent, separating and concentrating the organic layer, purifying by conventional methods such as chirography to obtain compounds of formula (7a) to (7g) as mentioned in the following table.

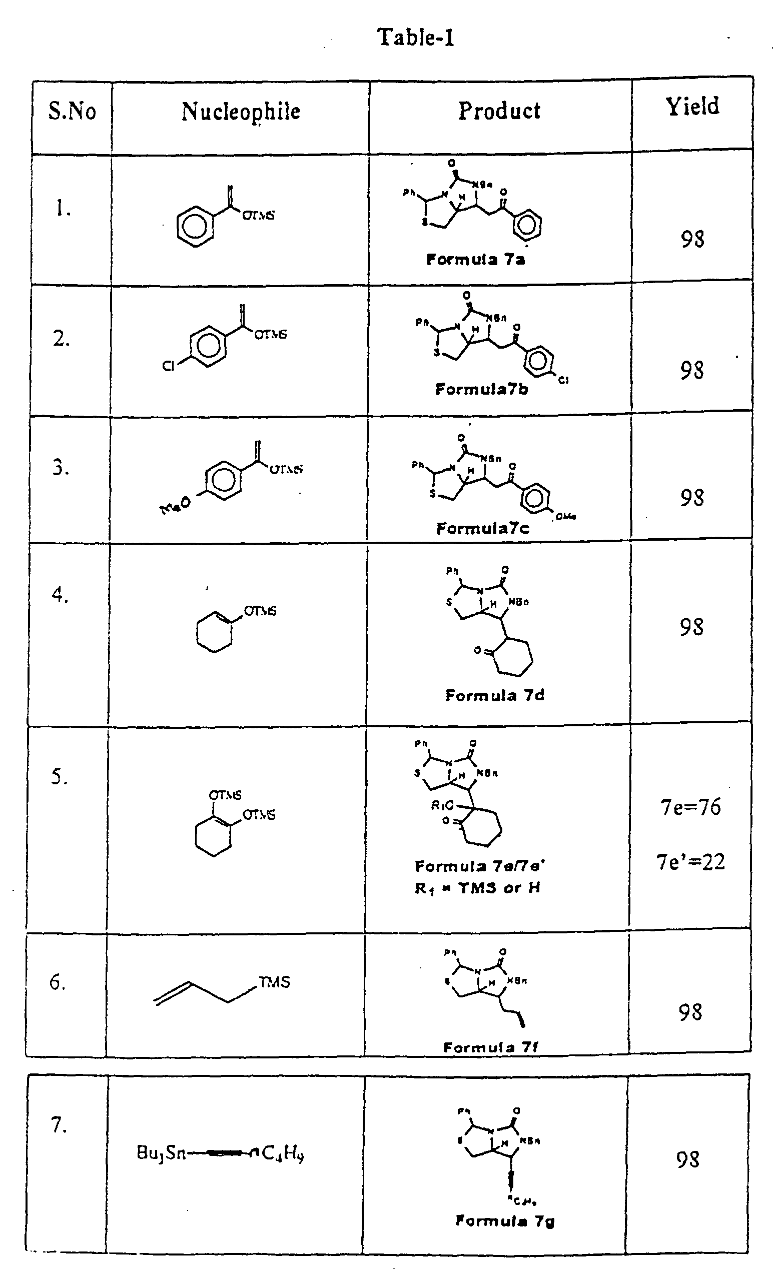

In one of the embodiment of the present invention the compound the alkyl group may be 1-phenyl -1-ethanone, 1-(4-chlorophenyl)-1-ethanone, 1-(4-methoxypheny)-1-ethanone,2-oxocyclohexyl, 1-trimethylsilyloxy-2-oxocyclohexyl, allyl, 1-hexanyl,4-dimethylaminophenyl, or 2-methylpropanoate.

In another embodiment the organic solvent used is selected from the group consisting of dichloromethane, dichloroethane, chloroform, tetrahydrofuran, benzene or toluene.

In still another embodiment the quenching agent is selected from the ammonium chloride, potassium chloride, sodium chloride.

The compound having formula (6) can be prepared by known procedures as per the scheme given herein below:

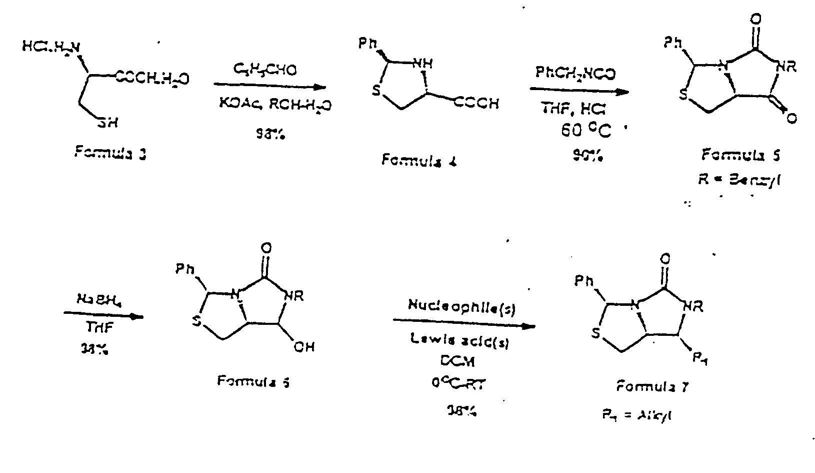

The present invention also provides a process for the preparation of D(+) biotin of formula (2) as per the scheme given herein below: 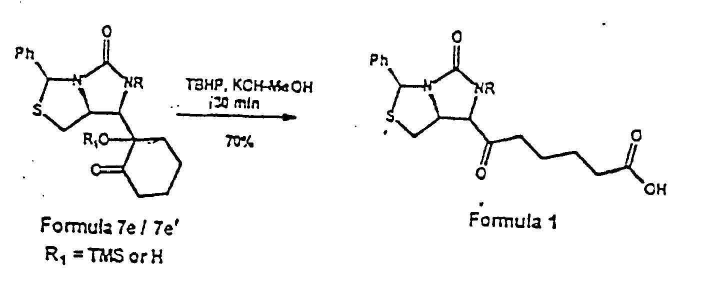 which comprises reacting compound having formula [7(e) / 7(e)] with an oxidizing agent in presence of alkaline alcohol at temperature ranging between 15-40°C for the period of 30 mints to 90 min, removing the alcohol by conventional methods and extracting with an organic solvent, separating the aqueous layer and acidifying to pH 4.0 to 5.0 further extracting the mixture with an solvent separating and concentrating the solvent layer, removing the solvent by conventional methods like evaporation to obtain compound of formula (1), converting the compound of formula (1) to obtain D(+) biotin by known methods.

In another embodiments of the present invention the solvent used for the reaction may be methanol, ethanol, water.

In another embodiment of the present invention the alkali used for the reaction may be KOH, NaOH, NaHCO₃, Na₂CO₃, NHCO₃, K₂CO₃.

In still another embodiment the oxidizing agent used maybe conventional peroixides exemplified by hydrogen peroxide, tertiary butyl hydrogen peroxide and cumene hydroperoxide etc.

In a feature of the present invention the electrophile of formula (6) is prepared by known procedure as given in (Poetsch, E.; Casutt, M., EP 242,686, 1986; CA: 108: 112077K 1988; *Chimia 41*, 141-150, 1987).

In another feature the present invention synthesis of D(+)-biotin can be done by obtaining methyl ether of compound having formula (6). The methyl ether can be prepared by conventional methods.

The merits of invented processes are use of easily accessible, cheap chemicals, selective Baeyer-Villiger oxidation and new methodology for the preparation of C-7 substituted imidazolidines, which provides an economically feasible method for the D(+)-biotin synthesis, as the synthesis of biotin of formula (2) from compound of formula (1) is well reported in literature, and avoids the use of costlier and hazardous chemicals such as DIBAL-H, KCN, etc.

As aforesaid D(+) biotin can be synthesized using compound mentioned in table-1. However one will have to follow different approaches for such synthesis.

### EXAMPLES

The process for the preparation of the compounds claimed in the present invention is described herein below which is illustrative only and should not be construed to limit the scope of the present invention in any manner whatsoever.

### EXAMPLE 1

### 2-[6-Benzyl-5-oxo-3-phenyl-(3S,7S,7aR)-perhydroimidazo[1,5-c][1,3]thiazol-7-yl]-1-phenyl-1-ethanone

To a solution of compound 6-benzyl-7-hydroxy-3-phenyl-(3S,7aR)-perhydroimidazo[1,5-C][1,3]thiazol-5-one of formula (6) (0.326 parts, 1mmol) in dichloromethane (10 parts) was added 1-trimethylsilyloxy styrene (0.384 parts, 2 mmol). Then the solution was cooled to 0°C, and Lewis acid for example borontrifluoride etherate (BF₃.Et₂O) (0.142 parts, I mmol) was added drop wise. The reaction mixture was stirred at 0°C for 10 mints, and the reaction mixture was quenched with saturated ammonium chloride (10 parts). Then the organic layer was separated, concentrated and column purification which ethylacetate:pet.ether (15:85) as eluent provided compound 2-[6-benzyl-5-oxo-3-phenyl-(3S, 7S, 7aR)-perhydroimidazo[1,5-C][1,3]thiazol-7-yl]-1-phenyl-1-ethanone of formula (7a) (viscous liquid, 0.419 parts, 0.98 nmol) in 98% yield.
¹H NMR (CDCl₃)δ: 2.64 (dd, 1H, J= 9.3, 10.3 Hz); 3.12 (dd, 1H, J= 4.4, 10.3Hz); 3.34 (m, 2H); 3.77 (m, 1H); 3.80 (m, 1H); 4.16 (d, 1H, J= 15.14 Hz); 4.86 (d, 1H, J= 15.14 Hz); 6.46 (s, 1H); 7.29 (m, 15H).

### EXAMPLE 2

### 2-[6-Benzyl-5-oxo-3-phenyl-(3S,7S,7aR)-perhydroimidazo[1,5-C][1,3]thiazol-7-yl]-1-(4-chlorophenyl)-1-ethanone

To a solution of compound 6-benzyl-7-hydroxy-3-phenyl-(3S,7aR)-perhydroimidazo[1,5-C][1,3]thiazol-5-one of formula (6) (0.326 parts, 1 mmol) in dichloromethane (10 parts) was added 1-trimethylsilyloxy styrene (0.453 parts, 2 mmol). Then the solution was cooled to 0°C, and Lewis acid for example BF₃.Et₂O (0.142 parts, 1 mmol) was added drop wise. The reaction mixture was stirred at 0°C for 15 mints, and the reaction mixture was quenched with saturated ammonium chloride (10 parts). Then the organic layer was separated, concentrated and column purification which ethylacetate:pet.ether (15:85) as eluent provided compound 2-[6-benzyl-5-oxo-3-phenyl-(3S, 7S, 7aR)-perhydroimidazo[1,5-C][1,3]thiazol-7-yl]-1-(4-chlorophenyl)-1-ethanone of formula (7b) (viscous liquid, 0.453 parts, 0.98 mmol) in 98% yield.
¹H NMR (CDCl₃)δ: 2.68 (dd, 1H, J= 9.3, 10.3 Hz); 3.09 (dd, 1H, J= 9.6, 17.5Hz); 3.31 (dd, 1H, J= 4.6, 10.3 Hz); 3.43 (dd, 1H, J= 3.9, 17.5 Hz); 3.75 (dd, 1H, J= 6.3, 9.1 Hz); 3.92 (dd, 1H, J= 3.9. 9.3 Hz); 4.16 (d, 1H, J= 15.14 Hz); 4.81 (d, 1H, J= 15.14 Hz); 6.45 (s. 1H): 7.28 (m, 10H); 7.39 (d, 2H, J= 8.79 Hz); 7.76 (d. 2H, J= 8.79 Hz).

### EXAMPLE 3

### 2-[6-Benzyl-5-oxo-3-phenyl-(3S,7S,7aR)-perhydroimidazo[1,5-C][1,3]thiazol-7-yl]-1-(4-methoxyphenyl)-1-ethanone

To a solution of compound 6-benzyl-7-hydroxy-3-phenyl-(3S,7aR)-perhydroimidazo[1,5-C][1,3]thiazol-5-one of formula (6) (0.326 parts, 1 mmol) in dichloromethane (10 parts) was added 1-trimethylsilyloxy 4'-methoxystyrene (0.444 parts, 2 mmol). Then the solution was cooled to 10°C, and Lewis acid for example BF₃.Et₂O (0.142 parts, 1 mmol) was added drop wise. The reaction mixture was stirred at 10°C for 20 mints, and the reaction mixture was quenched with saturated ammonium chloride (10 parts). Then the organic layer was separated, concentrated and column purification with ethylacetate:pet.ether (15:85) as eluent provided compound 2-[6-benzyl-5-oxo-3-phenyl-(3S, 7S, 7aR)-perhydroimidazo[1,5-C][1,3]thiazol-7-yl]-1-(4-methoxyphenyl)-1-ethanone of formula (7c) (viscous liquid, 0.449 parts, 0.98 mmol) in 98% yield.
¹H NMR (CDCl₃)δ: 2.67 (dd, 1H, J= 8.7, 10.3 Hz); 3.15 (dd, 1H, J= 9.4, 17.4Hz); 3.34 (m, 2H); 3.77 (m, 3H); 3.88 (s, 1H); 4.16 (d, 1H, J= 15.14 Hz); 4.86 (d, 1H, J= 15.14 Hz); 4.46 (s, 1H); 6.93 (d, 2H, J= 8.79): 7.31 (m, 10H); 7.83 (d, 2H, J= 8.79 Hz).

### EXAMPLE 4

### 6-Benzyl-7-[2-oxocyclohexyl)-3-phenyl-(3S,7S,7aR)-perhydroimidazo[1,5-C][1,3]thiazol-5-one

To a solution of compound 6-benzyl-7-hydroxy-3-phenyl-(3 S,7aR)-perhydroimidazo[1,5-C][1,3]thiazol-5-one of formula (6) (0.326 parts, 1 mmol) in dichloromethane (10 parts) was added 1-trimethylsilyloxy 1-trimethylsilyloxy-1-cyclohexene (.340 parts, 2 mmol). Then the solution was cooled to 20°C, and Lewis acid for example BF₃.Et₂O (0.142 parts, 1 mmol) was added drop wise. The reaction mixture was stirred at 20°C for 10 mints, and the reaction mixture was quenched with saturated ammonium chloride (10 parts). Then the organic layer was separated, concentrated and column purification with ethylacetate:pet.ether (15:85) as eluent provided compound 6-benzyl-7-(2-oxocyclohexyl)3-phenyl-(3S, 7S, 7aR)-perhydroimidazo[1,5-C][1,3]thiazol-5-one of formula (7d) (viscous liquid, 0.398 parts, 0.98 mmol) in 98% yield.
¹H NMR (CDCl₃)δ: 1.65 (m, 2H); 1.9-2.1 (m, 4H); 2.32 (5, 2H); 2.56 (ddd, 1H, J= 4.5, 6.7, 11.2 Hz); 3.33 (dd, 1H, J= 6, 10 Hz); 3.64 (ddd, 1H, J= 6.1, 7.6, 9 Hz); 3.87 )ddd, 1H, J= 2, 4.7, 12.3 Hz); 4.12 (d, 1H, J= 15.12 Hz); 4.70 (d, 1H, J= 15.12 Hz); 6.37 (s. 1H); 7.38 (m, 10H).

### EXAMPLE 5

### 6-Benzyl-7-[1-trimethylsilyloxy-2-oxocyclohexyl)-3-phenyl-(3S,7R, 7aR)-perhydroimidazo[1,5-C][1,3]thiazol-5-one

To a solution of compound 6-benzyl-7-hydroxy-3-phenyl-(3S,7aR)-perhydroimidazo[1,5-C][1,3]thiazol-5-one of formula (6) (0.326 parts, 1 mmol) in dichloromethane (10 parts) was added 1,2-bistrimethylsilyloxy cyclohexene (0.516 parts, 2 mmol). Then the solution was cooled to 0°C, and Lewis acid for example BF₃.Et₂O (0.142 parts, 1 mmol) was added drop wise. The reaction mixture was stirred at 0°C for 10 mints, and the reaction mixture was quenched with saturated ammonium chloride (10 parts). Then the organic layer was separated, concentrated and column purification with ethylacetate:pet.ether (15:85) as eluent provided compound 6-benyl-7-(1-trimethylsilyloxy-2-oxocyclohexyl)-3-phenyl-(3S, 7R, 7aR)-perhydroimidazo [1,5-C][1,3]thiazole-5-one of formula (7e) (viscous liquid, 0.376 parts, 0.76 mmol) in 76% yield. And with ethyl acetate:pet.ether (25:75) as eluent provided the compound 6-benzyl-7-(1-hydroxy-2-oxocyclohexyl)-3-phenyl-(3S, 7R, 7aR)-perhydroimidazo [1,5-C][1,3]thiazole-5-one of formula (7e') (highly viscous liquid 0.093 parts, 0.22 mmol) in 22% yield.
¹H NMR (CDCl₃)δ: 0.05 (s, 6H); 0.12 (s, 6H); 1.6-2.1 (m, 6H); 2.15-2.3 (m, 2H); 2.59 (m, 1H); 3.01 (dd, 1H, J= 6.1, 10.3 Hz); 3.89 (m, 2H); 4.13 (d, 1H, J= 15.14 Hz); 4.81 (d, 1H, J= 15.14 Hz); 6.48 (s, 1H); 7.38 (m, 10H).

### EXAMPLE 6

### 6-Benzyl-7-[1-trimethylsilyloxy-2-oxocyclohexyl)-3-phenyl-(3S, 7R, 7aR)-perhydroimidazo[1,5-C][1,3]thiazol-5-one

To a solution of compound 6-benzyl-7-hydroxy-3-phenyl-(3S,7aR)-perhydroimidazo[1,5-C][1,3]thiazol-5-one of formula (6) (0.340 parts, 1 mmol) in dichloromethane (10 parts) was added 1,2-bistrimethylsilyloxy cyclohexene (0.516 parts, 2 mmol). Then the solution was cooled to 0°C, and Lewis acid for example BF₃.Et₂O (0.142 parts, 1 mmol) was added drop wise. The reaction mixture was stirred at 0°C for 10 mints, and the reaction mixture was quenched with saturated ammonium chloride (10 parts). Then the organic layer was separated, concentrated and column purification with ethylacetate:pet.ether (15:85) as eluent provided compound 6-benyl-7-(1-trimethylsilyloxy-2-oxocyclohexyl)-3-phenyl-(3S, 7R, 7aR)-perhydroimidazo [1,5-C][1,3]thiazole-5-one of formula (7e) (viscous liquid, 0.376 parts, 0.76 mmol) in 76% yield. And with ethyl acetate:pet.ether (25:75) as eluent provided compound 6-benzyl-7-(1-trimethylsilyloxy-2-oxocyclohexyl)-3-phenyl-(3S, 7R, 7aR)-perhydroimidazo [1,5-C][1,3]thiazole-5-one of formula (7e) (viscous liquid, 0.490 parts, 0.98 mmol) in 98% yield.

### EXAMPLE 7

### 7-Allyl-6-benzyl-3-phenyl-(3S, 7S, 7aR)-perhydroimidazo[1,5-C][1,3]thiazol-5-one

To a solution of compound 6-benzyl-7-hydroxy-3-phenyl-(3S,7aR)-perhydroimidazo[1,5-C][1,3]thiazol-5-one of formula (6) (0.326 parts, 1 mmol) in dichloromethane (10 parts) was added allyltrimethylsilane (0.228 parts, 2 mmol). Then Lewis acid for example BF₃.Et₂O (0.142 parts, 1 mmol) was added drop wise to reaction mixture at 30°C was stirred 30 mints, and the reaction mixture was quenched with saturated ammonium chloride (10 parts). Then the organic layer was separated, concentrated and column purification with ethylacetate:pet.ether (15:85) as eluent provided compound 7-allyl-6-benzyl-3-phenyl-(3S, 7R, 7aR)-perhydroimidazo [1,5-C][1,3]thiazol-5-one of formula (7f) (viscous liquid, 0.343 parts, 0.98 mmol) in 98% yield.
¹H NMR (CDCl₃)δ: 2.4 (m, 2H); 2.55 (dd, 1H, J= 9, 10 Hz); 3.07 (dd, 1H, J= 6, 10 Hz); 3.35 (m, 1H); 3.80 (m, 1H); 4.08 (d, 1H, J= 15.14 Hz); 4.96 (d, 1H, J= 15.14 Hz); 5.18 (m, 2H); 5.76 (m, 1H); 6.46 (s, 1H); 7.32 (m, 10H).

### EXAMPLE 8

### 6-Benzyl-7-(1-hexynyl)-3-phenyl-(3S,7S,7aR)-perhydroimidazo[1,5-c][1,3]thiazol-5-one

To a solution of compound 6-benzyl-7-hydroxy-3-phenyl-(3S,7aR)-perhydroimidazo[1,5-C][1,3]thiazol-5-one of formula (6) (0.326 parts, 1 mmol) in dichloromethane (10 parts) was added 1-(tri n-butyltin)hexyne(0.742 parts, 2 mmol). Then the solution was cooled to 0°C, and Lewis acid for example BF₃.Et₂O (0.142 parts, 1 mmol) was added drop wise. The reaction mixture at 0°C for 10 mints, and the reaction mixture was quenched with saturated ammonium chloride (10 parts). Then the organic layer was separated, concentrated and column purification with ethylacetate:pet.ether (15:85) as eluent provided compound 6-benzyl-7-(1-hexynyl)-3-phenyl-(3S, 7R, 7aR)-perhydroimidazo[1,5-C][1,3]thiazol-5-one of formula (7g) (viscous liquid, 0.382 parts, 0.98 mmol) in 98% yield.
¹H NMR (CDCl₃)δ: 0.98 (t, 3H); 1.50(m, 4H); 2.27 (t, 2H); 2.62 (dd, 1H, J= 10.3 Hz); 3.12 (dd, 1H, J= 6.3, 10.4 Hz); 3.96 (d, 1H, J= 1.5 Hz); 4.06 (dd, 1H, 6.3, 9.0 Hz); 4.1 (d, 1H, J= 14.7 Hz); 5.0 (d, 1H, J= 14.7 Hz); 6.46 (s, 1H); 7.32 (m, 10H).

### EXAMPLE 9

### Methyl 6-[6-benzyl-5-oxo-3-phenyl-(3S, 7aR)-perhydroimidazo [1,5-C][1,3]-thiazol-7yl]-6-oxohexanoic acid

To an alkaline solution of methanol (0.168 parts, 3 mmol of KOH was dissolved in 10 parts of methanol) was added compound of formula (7e) (0.494 parts, 1 mmol). The solution was cooled to 0°C and then t-butylhydrogen peroxide (0.180 parts 2 mmol) was added in drop wise. The reaction mixture was stirred for an additional 30 mints. After 30 mints the methanol was removed and extracted with ethylacetate. The aqueous layer was acidified to ~ pH 3-4 and extracted with ethylacetate. Evaporation under reduced pressure furnished the compound process for 6-[6-benzyl-5-oxo-3-phenyl-(3S, 7aR)-perhydroimidazo[1,5-C][1,3]thiazol-7yl]-6-oxohexanoic acid of formula (1) (0.326 parts, 0.75 mmol) in 75% yield. This acid was characterized by its derivative as a methyl ester.
NMR (CDCl₃)δ: 1.55 (m, 4H); 2.27 (m, 2H); 2.44 (m, 2H); 2.58 (dd, 1H, J= 9.3, 10.3 Hz); 3.17 (dd, 1H, J= 6.3, 10.3 Hz); 3.66 (s, 3H); 3.75 (s, 1H); 3.80 (dd, 1H, J= 6.3, 9.0 Hz); 4.06 (d, 1H, J= 14.65 Hz); 4.98 (d, 1H, J= 14.65 Hz); 6.45 (s, 1H); 7.36 (m, 10H).

### EXAMPLE 10

### Methyl 6-[6-benzyl-5-oxo-3-phenyl-(3S, 7aR)-perhydroimidazo [1,5-C][1,3]-thiazol-7yl]-6-oxohexanoic acid

To an alkaline solution of methanol (0.168 parts, 3 mmol of KOH was dissolved in 10 parts of methanol) was added compound of formula (7e') (0.422 parts, 1 mmol). The solution was cooled to 0°C and then t-butylhydrogen peroxide (0.180 parts 2 mmol) was added in drop wise. The reaction mixture was stirred for an additional 30 mints. After 30 mints the methanol was removed and extracted with ethylacetate. The aqueous layer was acidified to ~ pH 3-4 and extracted with ethylacetate. Evaporation under reduced pressure furnished the compound process for 6-[6-benzyl-5-oxo-3-phenyl-(3S, 7aR)-perhydroimidazo[1,5-C][1,3]thiazol-7yl]-6-oxohexanoic acid of formula (1) (0.326 parts, 0.75 mmol) in 75% yield. This acid was characterized by its derivative as a methyl ester as mentioned in example 11.

### EXAMPLE 11

### Methyl 6-[6-benzyl-5-oxo-3-phenyl-(3S, 7aR)-perhydroimidazo [1,5-c][1,3]-thiazol-7yl]-6-oxohexanoic acid

To an alkaline solution of methanol (0.168 parts, 3 mmol of KOH was dissolved in 10 parts of methanol) was added compound of formula (7e) (0.494 parts, 1 mmol). The solution was cooled to 0°C and then hydrogen peroxide (0.128 parts 4 mmol) was added in drop wise. The reaction mixture was stirred for an additional 30 mints. After 30 mints the methanol was removed and extracted with ethylacetate. The aqueous layer was acidified to ~ pH 3-4 and extracted with ethylacetate. Evaporation under reduced pressure furnished the compound process for 6-[6-benzyl-5-oxo-3-phenyl-(3S, 7aR)-perhydroimidazo[1,5-C][1,3]thiazol-7yl]-6-oxohexanoic acid of formula (1) (0.196 parts, 0.45 mmol) in 45% yield. This acid was characterized by its derivative as a methyl ester as mentioned in example 11.

### EXAMPLE 12

### Methyl 6-[6-benzyl-5-oxo-3-phenyl-(3S,7aR)-perhydroimidazo [1,5-c][1,3]-thiazol-7yl]-6-oxohexanoic acid

To an alkaline solution of methanol (0.168 parts, 3 mmol of KOH was dissolved in 10 parts of methanol) was added compound of formula (7e) (0.494 parts, 1 mmol). The solution was cooled to 0°C and then cumenehydroperoxide (0.240 parts 2 mmol) was added in drop wise. The reaction mixture was stirred for an additional 30 mints. After 30 mints the methanol was removed and extracted with ethylacetate. The aqueous layer was acidified to ~ pH 3-4 and extracted with ethylacetate. Evaporation under reduced pressure furnished the compound process for 6-[6-benzyl-5-oxo-3-phenyl-(35, 7aR)-perhydroimidazo[1,5-C][1,3]thiazol-7yl]-6-oxohexanoic acid of formula (1) (0.260 parts, 0.60 mmol) in 60% yield. This acid was characterized by its derivative as a methyl ester as mentioned in example 11.

**The main advantages of the present invention are**:
1. Novelty
2. Simplicity
3. Ease of Operation
4. Short Reaction Time
5. Non-anhydrous conditions

Although the invention has been described in conjunction with specific embodiment, it is evident that many alternative and variations will be apparent to those skilled in the art in light of the foregoing description.

## Claims

1. A substituted 2-[-6-benzyl-5-oxo-3-phenyl-(3s,7s,7aR)-perhydroimidazol[1,5-c][1,3]thiazol] of formula (7) as shown herein below. 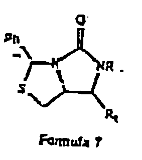 wherein R= benzyl, R₁ = alkyl group selected from 1-phenyl -1-ethanone, 1-(4-chlorophenyl)-1-ethanone, 1-(4-methoxypheny)-1-ethanone,2-oxocyclohexyl, 1-trimethylsilyloxy-2-oxocyclohexyl, allyl,1-hexanyl,4-dimethylaminophenyl, or 2-methylpropanoate.

2. The substituted 2-[-6-benzyl-5-oxo-3-phenyl-(3s,7s,7aR)-perhydroimidazol[1,5-c][1,3]thiazol] as claimed in claim 1 comprising compounds:
7a) 2-[6-benzyl-5-oxo-3-phenyl-(3S,7S,7aR)-perhydroimidazo[1,5-c][1,3]thiazol-7-yl]-1-phenyl ethanone,
7b) 2-[6-benzyl-5-oxo-3-phenyl-(3S,7S,7aR)-perhydroimidazo[1,5-c][1,3]thiazol-7-yl]-1-(4-chlorophenyl)-1-ethanone,
7c) 2-[6-benzyl-5-oxo-3-phenyl-(3S,7S,7aR)-perhydroimidazo[1,5-c][1,3]thiazol-7-yl]-1-(4-methoxyphenyl)-1-ethanone,
7d) 6 benzyl -7-(2-oxocyclohexyl)-3-phenyl-(3S,7S,7aR)-perhydroimidazo[1,5-c][1,3]thiazol-5-one,
7e) 6 benzyl -7-(1-trimethylsilyloxy-2-oxocyclohexyl)-3-phenyl-(3S,7S,7aR)-perhydroimidazo[1,5-c][1,3]thiazol-5-one,
7e') 6 benzyl -7-(1-hydroxy-2-oxocyclohexyl)-3-phenyl-(3S,7S,7aR)-perhydroimidazo[1,5-c][1,3]thiazol-5-one,
7f) 7-allyl-6-benzyl-3-phenyl-(3S, 7S, 7aR)- perhydroimidazo[1,5-c][1,3]thiazol-5-one, and
7g) 6-benzyl-7-(1-hexynyl)-3-phenyl(3S,7S,7aR)-perhydroimidazol[1,5c][1,3] thiazol - 5-one.

3. The substituted 2-[-6-benzyl-5-oxo-3-phenyl-(3s,7s,7aR)-perhydroimidazol[1,5-c][1,3]thiazol] as claimed in claim 1 being keto acids of formula 1.

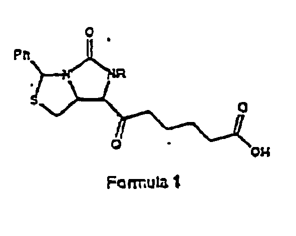

4. A process for the preparation of compounds having formula 7, 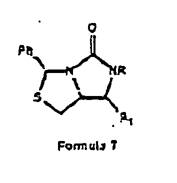 said process comprising reacting compound of formula 6 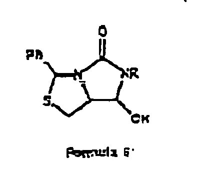 with Lewis acid and a nucleophile at a temperature ranging between 0° to 30 °C in an organic solvent for 10-30 minutes, quenching the reaction mixture with either water or saturated alkaline aqueous solution as quenching agent, separating and concentrating the organic layer, purifying by conventional methods such as chromatography to obtain compound of formula (7).

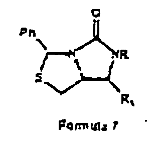

5. A process as claimed in claim 4, wherein the nucleophile is selected from the group consisting of 1-phenyl -1-ethanone, 1-(4-chlorophenyl)-1-ethanone, 1-(4-methoxypheny)-1-ethanone, 2-oxocyclohexyl, 1-trimethylsilyloxy-2-oxocyclohexyl, allyl, 1-hexanyl, 4-dimethylaminophenyl, and 2-methylpropanoate.

6. A process as claimed in claim 4, wherein the organic solvent used is selected from the group consisting ofdichloromethane, dichloroethane, chloroform, tetrahydrofuran, benzene and toluene.

7. A process as claimed in claim 4, wherein the quenching agent is selected from the ammonium chloride, potassium chloride, and sodium chloride.

8. A process for the preparation of keto acid of formula (1), 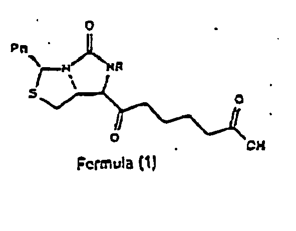 said process comprising reacting a compound having formula 7(e) or 7(e')
7e) 6 benzyl-7-(1-trimethylsilyloxy-2-oxocyclohexyl)-3-phenyl-(3S, 7S, 7aR)-perhydroimidazo [1,5-C][1,3]thiazol-5-one,
7e') 6 benzyl-7-(1-hydroxy-2-oxocyclohexyl)-3-phenyl-(3S,7S,7aR)-perhydroimidazo[1,5-C][1,3]thiazol-5-one,
with an oxidizing agent in presence of alkaline alcohol at temperature ranging between 15-40°C for the period of 30-90 min, removing the alcohol by conventional methods and extracting with an organic solvent, separating the aqueous layer and acidifying to pH 4.0 to 5.0, further extracting the mixture with an solvent, separating and concentrating the solvent layer, removing the solvent by conventional methods like evaporation to obtain compound of formula (1), and optionally, converting the compound of formula (1) to obtain D(+).

9. A process as claimed in claim 8, wherein the solvent is selected from water, ethanol, and methanol.

10. A process as claimed in claim 8 wherein the alkali used in the reaction is selected from the group consisting of KOH, NaOH, NaHCO₃, Na₂CO₃, KHCO₃ and K₂CO₃.

11. A process as claimed in claim 8 wherein, the oxidizing agents used are conventional peroxides selected from hydrogen peroxide, tertiary butyl hydrogen peroxide and cumene hydro peroxide.

12. A process as claimed in claim 8 wherein, the organic layer was separated, concentrated and column purification with ethylacetate: petroleum ether (15:85) as eluent.

## Patentansprüche

1. Ein substituiertes 2-[6-Benzyl-5-oxo-3-phenyl-(3S,7S,7aR)-perhydroimidazol[1,5-c][1,3]thiazol] der Formel (7), wie hier unten gezeigt 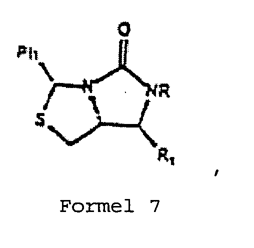 wobei R = Benzyl, R₁ = Alkylgruppe, die aus 1-Phenyl-1-ethanon, 1-(4-Chlorphenyl)-1-ethanon, 1-(4-Methoxyphenyl)-1-ethanon, 2-Oxocyclohexyl, 1-Trimethylsilyloxy-2-oxocyclohexyl, Allyl, 1-Hexanyl, 4-Dimethylaminophenyl oder 2-Methylpropanoat ausgewählt wird.

2. Das substituierte 2-[6-Benzyl-5-oxo-3-phenyl-(3S,7S,7aR)-perhydroimidazol[1,5-c][1,3]thiazol] wie in Anspruch 1 beansprucht, das die Verbindungen umfasst:
7a) 2-[6-Benzyl-5-oxo-3-phenyl-(3S,7S,7aR)-perhydroimidazo[1,5-c][1,3]thiazol-7-yl]-1-phenylethanon,
7b) 2-[6-Benzyl-5-oxo-3-phenyl-(3S,7S,7aR)-perhydroimidazo[1,5-c][1,3]thiazol-7-yl]-1-(4-chlorophenyl)-1-ethanon,
7c) 2-[6-Benzyl-5-oxo-3-phenyl-(3S,7S,7aR)-perhydroimidazo[1,5-c][1,3]thiazol-7-yl]-1-(4-methoxyphenyl)-1-ethanon,
7d) 6-Benzyl-7-(2-oxocyclohexyl)-3-phenyl-(3S,7S,7aR)-perhydroimidazo[1,5-c][1,3]thiazol-5-on,
7e) 6-Benzyl-7-(1-trimethylsilyloxy-2-oxocyclohexyl)-3-phenyl-(3S,7S,7aR)-perhydroimidazol[1,5-c][1,3]-thiazol-5-on,
7e') 6-Benzyl-7-(1-hydroxy-2-oxocyclohexyl)-3-phenyl-(3S,7S,7aR)-perhydroimidazo[1,5-c][1,3]thiazol-5-on,
7f) 7-Allyl-6-benzyl-3-phenyl-(3S,7S,7aR)-perhydroimidazo[1,5-c][1,3]thiazol-5-on und
7g) 6-Benzyl-7-(1-hexynyl)3-phenyl-(3S,7S,7aR)-perhydroimidazo[1,5-c][1,3]thiazol-5-on.

3. Das substituierte 2-[6-Benzyl-5-oxo-3-phenyl-(3S,7S,7aR)-perhydroimidazol[1,5-c][1,3]thiazol] wie in Anspruch 1 beansprucht, das Ketosäure der Formel 1 ist

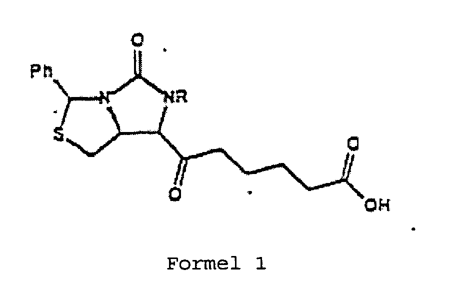

4. Ein Verfahren zur Herstellung von Verbindungen mit der Formel 7 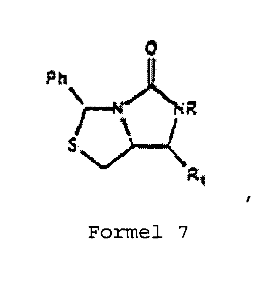 wobei das Verfahren das Umsetzen einer Verbindung von Formel 6 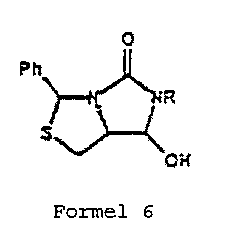 mit einer Lewis-Säure und einem Nukleophil bei einer Temperatur, die im einem Bereich zwischen 0° bis 30°C liegt, in einem organischen Lösungsmittel für 10-30 Minuten, das Quenchen der Reaktionsmischung entweder mit Wasser oder gesättigter wässriger basischer Lösung als Quenchmittel, das Trennen und Konzentrieren der organischen Schicht, das Reinigen mittels herkömmlicher Verfahren, wie z.B. Chromatographie, um eine Verbindung der Formel 7 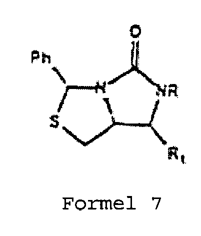 zu erhalten, umfasst.

5. Ein Verfahren wie in Anspruch 4 beansprucht, wobei das Nukleophil aus der Gruppe, die aus 1-Phenyl-1-ethanon, 1-(4-Chlorphenyl)-1-ethanon, 1-(4-Methoxyphenyl)-1-ethanon, 2-Oxocyclohexyl, 1-Trimethylsilyloxy-2-oxocyclohexyl, Allyl, 1-Hexanyl, 4-Dimethylaminophenyl oder 2-Methylpropanoat besteht, ausgewählt wird.

6. Ein Verfahren wie in Anspruch 4 beansprucht, wobei das verwendete organische Lösungsmittel aus der Gruppe, die aus Dichlormethan, Dichlorethan, Chloroform, Tetrahydrofuran, Benzol und Toluol besteht, ausgewählt wird.

7. Ein Verfahren wie in Anspruch 4 beansprucht, wobei das Quenchmittel aus Ammoniumchlorid, Kaliumchlorid und Natriumchlorid ausgewählt wird.

8. Ein Verfahren zur Herstellung einer Ketosäure der Formel (1) 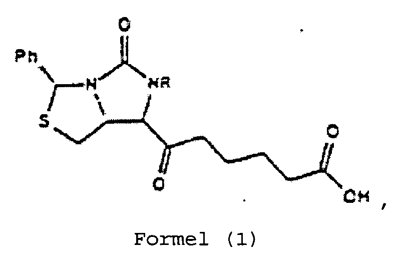 wobei das Verfahren das Umsetzen einer Verbindung mit Formel 7(e) oder 7(e')
7e) 6-Benzyl-7-(1-trimethylsilyloxy-2-oxocyclohexyl)-3-phenyl-(3S,7S,7aR)-perhydroimidazo[1,5-c][1,3]thiazol-5-on,
7e') 6-Benzyl-7-(1-hydroxy-2-oxocyclohexyl)-3-phenyl-(3S,7S,7aR)-perhydroimidazol[1,5-c][1,3]thiazol-5-on
mit einem Oxidationsmittel in Gegenwart eines basischen Alkohols bei einer Temperatur, die in einem Bereich zwischen 15-40°C liegt, über den Zeitraum von 30-90 min, das Entfernen des Alkohols mit herkömmlichen Verfahren und das Extrahieren mit einem organischen Lösungsmittel, das Trennen der wässrigen Schicht und das Ansäuern auf pH 4.0 bis 5.0, des Weiteren das Extrahieren der Mischung mit einem Lösungsmittel, das Trennen und das Konzentrieren der Lösungsmittelschicht, das Entfernen des Lösungsmittels mit herkömmlichen Verfahren, wie Verdampfung, um eine Verbindung der Formel (1) zu erhalten, und gegebenenfalls das Umwandeln der Verbindung nach Formel (1), um D(+) zu erhalten, umfasst.

9. Ein Verfahren wie in Anspruch 8 beansprucht, wobei das Lösungsmittel aus Wasser, Ethanol und Methanol ausgewählt wird.

10. Ein Verfahren wie in Anspruch 8 beansprucht, wobei das bei der Reaktion verwendete Alkali aus der Gruppe, die aus KOH, NaOH, NaHCO₃, Na₂CO₃, KHCO₃ und K₂CO₃ besteht, ausgewählt wird.

11. Ein Verfahren wie in Anspruch 8 beansprucht, wobei die verwendeten Oxidationsmittel herkömmliche Peroxide sind, die aus Wasserstoffperoxid, Tertiär-Butyl Wasserstoffperoxid und Cumolhydroperoxid ausgewählt werden.

12. Ein Verfahren wie in Anspruch 8 beansprucht, wobei die organische Schicht getrennt, konzentriert und mit Ethylacetat:Petrolether (15:85) als Eluent mittels einer Säule gereinigt wird.

## Revendications

1. Le 2-[6-benzyl-5-oxo-3-phényl-(3S,7S,7aR)-perhydroimidazol[1,5-c][1,3]thiazol] substitué de formule (7) représentée ci-dessous, 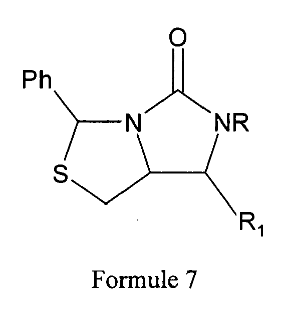 dans laquelle R = benzyle, R₁ = groupe alkyle choisi parmi la 1-phényl-1-éthanone, la 1-(4-chlorophényl)-1-éthanone, la 1-(4-méthoxyphényl)-1-éthanone, le 2-oxocyclohexyle, le 1-triméthylsilyloxy-2-oxocyclohexyle, l'allyle, le 1-hexanyle, le 4-diméthylaminophényle, ou le 2-méthylpropanoate.

2. Le 2-[6-benzyl-5-oxo-3-phényl-(3S,7S,7aR)-perhydroimidazol[1,5-c][1,3]thiazol] substitué selon la revendication 1 comprenant les composés :
7a) 2-[6-benzyl-5-oxo-3-phényl-(3S,7S,7aR)-perhydroimidazo[1,5-c][1,3]thiazol-7-yl]-1-phényl éthanone,
7b) 2-[6-benzyl-5-oxo-3-phényl-(3S,7S,7aR)-perhydroimidazo[1,5-c][1,3]thiazol-7-yl]-1-(4-chlorophényl)-1-éthanone,
7c) 2-[6-benzyl-5-oxo-3-phényl-(3S,7S,7aR)-perhydroimidazo[1,5-c][1,3]thiazol-7-yl]-1-(4-méthoxyphényl)-1-éthanone,
7d) 6-benzyl-7-(2-oxocyclohexyl)-3-phényl-(3S,7S,7aR)-perhydroimidazo[1,5-c] [1,3]thiazol-5-one,
7e) 6-benzyl-7-(1-triméthylsilyloxy-2-oxocyclohexyl)-3-phényl-(3S,7S,7aR)-perhydroimidazo[1,5-c][1,3]thiazol-5-one,
7e') 6-benzyl-7-(1-hydroxy-2-oxocyclohexyl)-3-phényl-(3S,7S,7aR)-perhydroimidazo[1 ,5-c][1,3]thiazol-5-one,
7f) 7-allyl-6-benzyl-3-phényl-(3S,7S,7aR)-perhydroimidazo[1,5-c][1,3]thiazol-5-one, et
7g) 6-benzyl-7-(1-hexynyl)-3-phényl-(3S,7S,7aR)-perhydroimidazol[1,5-c][1,3]thiazol-5-one.

3. Le 2-[6-benzyl-5-oxo-3-phényl-(3S,7S,7aR)-perhydroimidazol[1,5-c][1,3]thiazol] substitué selon la revendication 1 qui est le céto acide de formule 1,

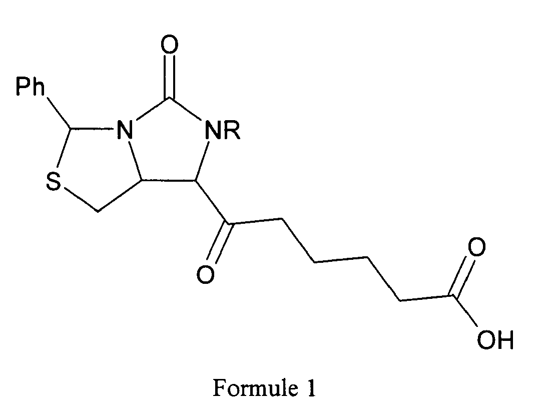

4. Procédé de préparation de composés de formule 7, 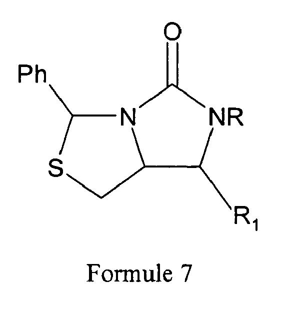 ledit procédé comprenant la réaction du composé de formule 6 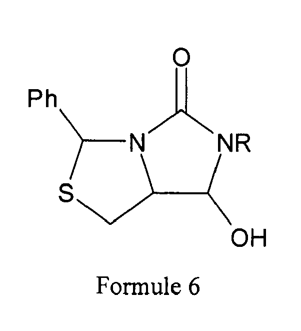 avec un acide de Lewis et un nucléophile à une température allant de 0°C à 30°C dans un solvant organique pendant 10 à 30 minutes, le traitement du mélange réactionnel avec de l'eau ou avec une solution aqueuse alcaline saturée utilisée comme agent de traitement, la séparation et la concentration de la phase organique, la purification par des procédés traditionnels tels que la chromatographie pour obtenir un composé de formule (7).

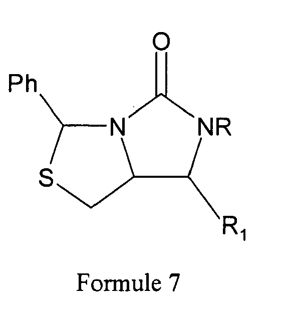

5. Procédé selon la revendication 4, dans lequel le nucléophile est choisi dans le groupe constitué par la 1-phényl-1-éthanone, la 1-(4-chlorophényl)-1-éthanone, la 1-(4-méthoxyphényl)-1-éthanone, le 2-oxocyclohexyle, le 1-triméthylsilyloxy-2-oxocyclohexyle, l'allyle, le 1-hexanyle, le 4-diméthylaminophényle et le 2-méthylpropanoate.

6. Procédé selon la revendication 4, dans lequel le solvant organique utilisé est choisi dans le groupe constitué par le dichlorométhane, le dichloroéthane, le chloroforme, le tétrahydrofuranne, le benzène et le toluène.

7. Procédé selon la revendication 4, dans lequel l'agent de traitement est choisi parmi le chlorure d'ammonium, le chlorure de potassium, et le chlorure de sodium.

8. Procédé de préparation d'un céto acide de formule (1), 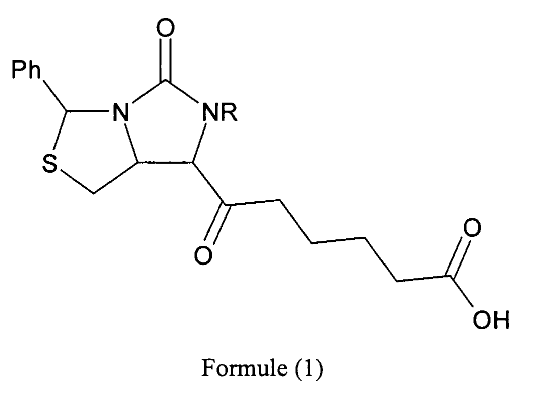 ledit procédé comprenant la réaction d'un composé de formule 7(e) ou 7(e') 7e) 6-benzyl-7-(1-triméthylsilyloxy-2-oxocyclohexyl)-3-phényl-(3S,7S,7aR)-perhydroimidazo[1,5-c][1,3]thiazol-5-one,
7e') 6-benzyl-7-(1-hydroxy-2-oxocyclohexyl)-3-phényl-(3S,7S,7aR)-perhydroimidazo[1,5-c][1,3]thiazol-5-one,
avec un agent oxydant en présence d'un alcool alcalin à une température allant de 15 à 40°C pendant 30 à 90 minutes, l'élimination de l'alcool selon des procédés traditionnels et l'extraction avec un solvant organique, la séparation de la phase aqueuse et l'acidification jusqu'à un pH de 4,0 à 5,0, une extraction supplémentaire du mélange avec un solvant, la séparation et la concentration de la phase de solvant, l'élimination du solvant par des procédés traditionnels comme l'évaporation pour obtenir un composé de formule (1), et facultativement, la conversion du composé de formule (1) pour obtenir un D(+).

9. Procédé selon la revendication 8, dans lequel le solvant est choisi parmi l'eau, l'éthanol, et le méthanol.

10. Procédé selon la revendication 8, dans lequel l'alcalin utilisé dans la réaction est choisi dans le groupe constitué par KOH, NaOH, NaHCO₃, Na₂CO₃, KHCO₃, et K₂CO₃.

11. Procédé selon la revendication 8, dans lequel les agents oxydants utilisés sont des peroxydes traditionnels choisis parmi le peroxyde d'hydrogène, le peroxyde d'hydrogène de butyle tertiaire et l'hydroperoxyde de cumène.

12. Procédé selon la revendication 8, dans lequel la phase organique a été séparée, concentrée et purifiée sur colonne avec, comme éluant, le mélange acétate d'éthyle:éther de pétrole (15 : 85).
